(19) Europäisches Patentamt
European Patent Office
Office européen des brevets

(11) Veröffentlichungsnummer: 0 104 584
B1

(12) **EUROPÄISCHE PATENTSCHRIFT**

(45) Veröffentlichungstag der Patentschrift: 03.09.86

(21) Anmeldenummer: 83109298.6

(22) Anmeldetag: 20.09.83

(51) Int. Cl.⁴: C 07 C 102/00, C 07 C 102/06, C 07 C 103/58, C 07 D 493/08 // (C07D493/08, 307:00, 307:00)

(54) **Verfahren zur Herstellung von N-substituierten Acrylsäureamiden.**

(30) Priorität: 24.09.82 DE 3235398

(43) Veröffentlichungstag der Anmeldung: 04.04.84 Patentblatt 84/14

(45) Bekanntmachung des Hinweises auf die Patenterteilung: 03.09.86 Patentblatt 86/36

(84) Benannte Vertragsstaaten: AT BE CH DE FR GB IT LI LU NL SE

(56) Entgegenhaltungen:
FR-A-2 136 357
FR-A-2 204 619

(73) Patentinhaber: Chemische Fabrik Stockhausen GmbH, Bäkerpfad 25, D-4150 Krefeld (DE)

(72) Erfinder: Landscheidt, Alfons, Dr. Dipl. Chem., Kölner Strasse 390, D-4150 Krefeld (DE)

(74) Vertreter: Klöpsch, Gerald, Dr.- Ing., An Gross St. Martin 6, D-5000 Köln 1 (DE)

EP 0 104 584 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von N-substituierten Acrylsäureamiden.

N-substituierte Acrylsäureamide sind Monomere mit einer Kohlenstoffdoppelbindung, die sich durch eine hohe Polymerisationsaktivität auszeichnet und besitzen als Säureamide eine hydrophile und zugleich hydrolysestabile funktionelle Gruppe. Diese Monomere sind deshalb von erheblichem technischen Interesse.

Zur Herstellung anderer Acrylsäurederivate werden aus wirtschaftlichen Überlegungen bevorzugt die niederen Ester der Acrylsäure eingesetzt. Auch N-substituierte Amide sind auf dieser Basis zugänglich. Dieses gelingt jedoch nicht durch eine direkte Umsetzung von Acrylsäureestern mit Aminen. Statt der erwarteten Esteramidierung tritt zuerst eine Addition des Amins an die aktivierte Kohlenstoffdoppelbindung im Sinne einer Michael-Reaktion ein. Erst das zweite Mol Amin setzt sich dann mit dem Ester zu N-substituierten 3-Aminopropionsäureamiden um. Diese können pyrolytisch zu den entsprechenden N-substituierten Acrylsäurederivaten gespalten werden (US 2,719,178).

Die Addition eines Amins an die Kohlenstoffdoppelbindung eines Acrylsäureesters ist demnach gegenüber der Esteramidierung stark bevorzugt. Erst bei drastisch höheren Temperaturen tritt eine Umkehrung der Reaktivitäten ein, da die Michael-Reaktion dann reversibel wird. Aus diesem Gleichgewicht heraus kann das Amin irreversibel mit der Estergruppe reagieren (US 2,719,175).

Wenn man die dazu nötige thermische Belastung und die damit verbundene mangelnde Reinheit der Monomeren nicht in Kauf nehmen will, ist man gezwungen, die Kohlenstoffdoppelbindung des Acrylsäureesters mit einer Schutzgruppe umzusetzen, bevor die Amidierung des Esters durchgeführt wird. Dieses kann wie im obigen Fall durch die Addition eines Amins geschehen, man kann jedoch auch andere protonenaktive Stoffe wie Halogenwasserstoffe, Carbonsäuren oder Alkohole an die Kohlenstoffdoppelbindung addieren und diese Doppelbindungsschutzgruppen nach erfolgter Esteramidierung unter geeigneten Reaktionsbedingungen wieder abspalten.

Eine Schutzgruppe anderer Art wird in der DAS 22 17 623 beschrieben. Hier werden Acrylsäurederivate mit Cyclopentadien in einer Diels-Alder-Reaktion zu Norbornensäurederivate umgesetzt. Das durch Umsetzung mit Aminen im zweiten Schritt erhaltene N-substituierte Norbonensäureamid wird durch Erhitzen auf 250°C in Cyclopentadien und das N-substituierte Acrylsäureamid gespalten. Auch bei dieser Synthese sind die Temperaturen für einige besonders empfindliche Monomere zu hoch, um die gewünschte Reinheit zu erzielen.

In der DE-OS 23 54 602 wird die Herstellung von basisch modifizierten N-substituierten Acrylamiden nach dem oben erwähnten Verfahren beschrieben. Auch diese Produkte werden nicht in der erforderlichen Reinheit erhalten, die bei Nsubstituierten Acrylamide, die eine tertiäre Amingruppe enthalten, erforderlich ist.

Monomere dieser Art werden bevorzugt zu hochmolekularen, linearen wasserlöslichen Polymeren umgesetzt. Anteile von nur 10 ppm an bifuktionellen Monomeren können hierbei Vernetzungen bewirken, die die Produktqualität der wasserlöslichen Polymere stark beeinträchtigen. So kann z.B. aus N(N',N'-Dimethylaminopropyl)acrylamid durch thermische Abspaltung von Dimethylamin N-Allylacrylamid entstehen, analog ist die Bildung von N-Vinylacrylamid aus N(N',N'-Dialkylaminoethyl)acrylamid möglich. Die Bildung dieser vernetzenden Monomere muß soweit wie möglich unterdrückt werden, um Monomere in einer Reinheit zu erhalten, die lineare, gelfreiwasserlösliche Polymere mit Molekulargewichten von über 10 Millionen ermöglicht. Dies kann bei der Herstellung von aminogruppenhaltigen Monomeren nur durch Anwendung schonender Reaktionsbedingungen, d.h. niedriger Temperaturen, geschehen.

Aufgabe der Erfindung ist daher ein Herstellungsverfahren für hochreine, N-substituierte Acrylsäureamide, die im wesentlichen frei sind von störenden bifunktionellen Monomeren, welche anschliessend bei der Polymerisation der N-substituierten Acrylsäureamide durch Vernetzung zur Bildung von unerwünschtem unlöslichen Produkt führen würden.

Überraschenderweise wurde gefunden, daß 2-Carboalkoxy-7-oxabicyclo(2,2,l)hept-5-ene, die durch eine Diels-Alder-Reaktion von Furan mit Acrylsäureestern leicht zugänglich sind, mit primären oder sekundären Aminen unter Basenkatalyse zu 2-Carboxamid-7-oxabicyclo(2,2,l)hept-5-enen umgesetzt werden können und durch Erhitzen nach Abspaltung von Furan N-substituierte Acrylsäureamide der geforderten Reinheit gemäß folgendem Formelschema ergeben:

I) 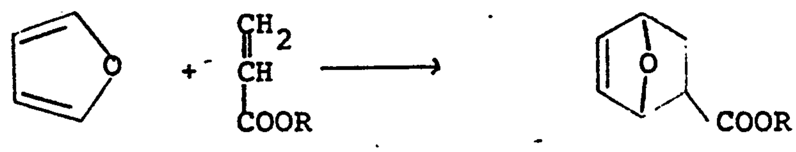

II) 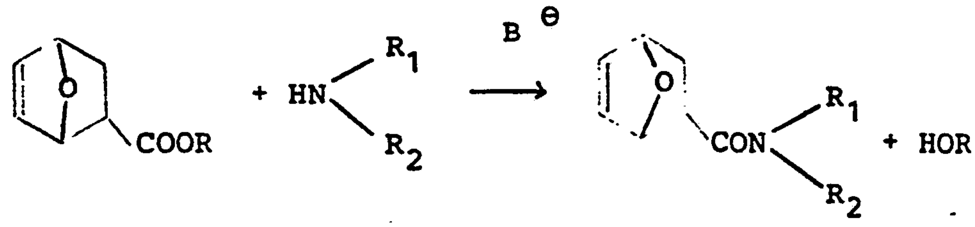


III) 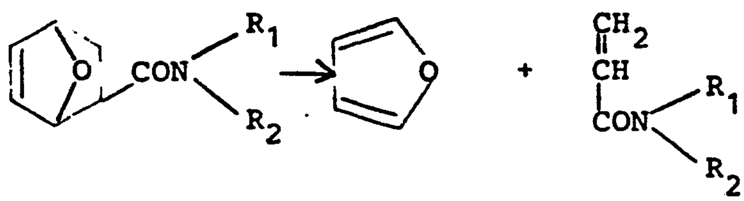

Zu erwarten wäre gewesen, daß sich unter dem Einfluß von Basen 7-Oxabicyclo(2,2,1)hept-5-ene in Benzolderivate umlagern. So bildet sich nach Helvetica Chimica Acta, Vol. 58 (1975), 1181 aus dem Diels-Alder-Addukt von 2-Methyl-furan und Acrylnitril beim Behandeln mit Kalium-t-butylat ein Gemisch aus 10 Teilen O-Tolunitril und 1 Teil m-Tolu nitril.

Die Aufgabe gemäß der Erfindung wird dadurch gelöst, daß man 2-Carboalkoxy-7-oxabicyclo(2,2,1)hept-5-ene mit primären oder sekundären Aminen zu 2-Carboxamid-7-oxabicyclo-(2,2,1)hept-5-enen umsetzt und diese unter Bildung von Furan und N-subtituierten Acrylsäureamiden zersetzt.

Die Amidierung der 2-Carboalkoxy-7-oxabicyclo(2,2,1)hept-5-ene erfolgt zweckmäßigerweise bei Temperaturen, die unterhalb der Zersetzungstemperatur des 7-oxabicyclo(2,2,1)-hept-5-en - Systems liegen. Bevorzugt werden Temperaturen zwischen 20° C und 60° C. Um bei diesen Temperaturen eine Esteramidierung zu erreichen, sind in der Regel wasserfreie Bedingungen und starke Basen erforderlich. Als Basen bevorzugt werden Alkalimetallalkoholate, -hydride oder -amide wie Natriummethylat, Natriumethylat, Natriumhydrid oder Kalium-t-butylat. Möglich ist allerdings auch der Einsatz von basischen Ionenaustauschern oder eine Esteramidierung unter den Bedingungen einer Phasentransferkatalyse.

Nach erfolgter Amidierung wird der Katalysator abgetrennt bzw. neutralisiert und das entstandene Salz durch Zusatz von Wasser abgetrennt. Das in der ersten Stufe der Reaktion entstandene N-subtituierte 2-Carboxamid-7-oxabicyclo-(2,2,1)hept-5-en wird dann thermisch zu Furan und N-substituierten Acrylsäureamid zersetzt. Diese Reaktion ist in starkem Maße temperaturabhängig. Schon bei Raumtemperatur tritt im Verlauf einiger Wochen eine teilweise Zersetzung ein. Ohne Kalatysatoren ist es jedoch notwendig, zur Erzielung von ausreichend kurzen Reaktionszeiten auf vergleichsweise hohe Temperaturen zwischen 60 und 300° C, vorzugsweise zwischen 80 und 200° C, insbesondere zwischen 100 und 150° C zu erhitzen. Durch Zusatz von Lewis-Säuren wie $AlCl_3$, $TiCl_4$, $SnCl_4$, $FeCl_3$ oder $BF_3.OET_2$ kann die Furan-Abspaltung bei Raumtemperatur wesentlich beschleunigt werden. Die Zersetzung läuft dann bereits bei Temperaturen zwischen 0 und 40° C, vorzugsweise zwischen und 25° C ab. Die Zersetzung wird bevorzugt durch Erhitzen im Vakuum vorgenommen.

Als Amine können primäre oder sekundäre Amine verwendet werden. Geeignet sind aliphatische gerad- oder verzweigtkettige primäre oder sekundäre Amine ebenso wie cyclische, aliphatische, heterocyclische oder aromatische Amine, die gegebenenfalls weitere funktionelle Gruppen tragen. Genannt seien aliphatische Amine, wie Methylamin, Ethylamin, propylamin, Isopropylamin, Butylamin, Isobutylamin, 2-Ethylhexylamin, Stearylamin als Beispiele für primäre Monoamine, Dimethylamin, Diethylamin als Beispiel für sekundäre Amine, Ethylendiamin, 1,3-propylendiamin und 1,6-Hexylendiamin als primäre Diamine, Morpholin, Pyrrolidin, Piperidin als Beispiele für heterocyclische Amine und Anilin, Toluidin, Anisidin und N-Methylanilin als Beispiele für aromatische Amine.

Besonders geeignet ist das Verfahren für primäre oder sekundäre Amine, die mindestens eine weitere sekundäre oder tertiäre Aminogruppe enthalten. Hier sind zu nennen N,N-Dimethylaminoethylamin, N,N-Diethylaminoethylamin, N,N-Dipropylaminoethylamin, N-Ethylaminoethylamin, N-Isopropylaminoethylamin, N-t-Butylaminoethylamin, N,N-Dimethylaminopropylamin, N,N,2,2-Tetramethyl-1,3-diaminopro-pan, N,N-Dimethylaminopropylamin, N,N-Diethylaminopropylamin, N-Methyl-piperazin und N-Methyl-N'(2-Aminoethyl)piperazin.

Die Zersetzungstemperaturen liegen dann, wenn die Zersetzung ohne Lewis-Säure als Katalysator verwendet wird, in den meisten Fällen unter den Siedetemperaturen der N-substituierten Acrylsäureamide bei technisch üblichem Vakuum. Die größte thermische Belastung der Syntheseprodukte tritt deshalb bei der abschliessenden Destillation auf. Die eigentliche Synthese wird bei niedrigeren Temperaturen durchgeführt. Speziell die Bildung von Vernetzern aus N-substituierten Acrylamiden, die eine Aminogruppe enthalten, wird durch diese Arbeitsweise unterdrückt. Aber auch die Reinheit weiterer nach den erfindungsgemäßen Verfahren hergestellter Monomerer wird durch die schonenden Reaktionsbedingungen positiv beeinflußt.

Die Erfindung wird durch die folgenden Beispiele erläutert:

**Beispiel 1:**

88 Teile N,N-Dimethylaminoethylamin werden mit 2,4 Teilen 80%igem Natriumhydrid in Paraffinöl versetzt. 154 Teile 2-Carbomethoxy-7-oxablcyclo(2,2,l)hept-5-en werden bei 20 bis 25°C unter Kühlen zugetropft. Man lässt 15 Stumden nachrühren und neutralisiert mit 8 Teilen konzentrierter Salzsäure. Man versetzt mit 200 Teilen Methylenchlorid und 100 Teilen Wasser, trennt die organische Phase ab und extrahiert die wäßrige Phase 4 mal mit je 50 Teilen Methylenchlorid. Die vereinigten organischen Phasen werden am Rotationsverdampfer bei einer Badtemperatur von 90°C und einem Vakuum Von 2700 Pa eingeengt. Der Rückstand wird im Rochvakuum auf 110 bis 120°C erhitzt (Badtemperatur 140°C). Nach Beendigung der Furan-Abspaltung steigt die Sumpftemperatur und das Produkt destilliert über Man erhält 106 Teile N-(N',N'-Dimethylaminoethyl)-acrylamid vom Siedepunkt 100°C/100 Pa.

**Beispiel 2:**

121,8 Teile N,N-Diethylaminoethylamin werden mit 9 Teilen Kalium-t-butylat versetzt. 154 Teile 2-Carbomethoxy-7-oxabicyclo(2,2,1)hept-5-en werden bei 20 bis 25°C unter Kühlen zugetropft. Man lässt 15 Stunden nachrühren und neutralisiert mit 8 Teilen konzentrierter Salzsäure. Man Versetzt mit 200 Teilen Methylenchlorid und 100 Teilen Wasser, trennt die organische Phase ab und extrahiert die wäßrige Phase 4 mal mit je 50 Teilen Methylenchlorid. Die vereinigten organischen Phasen werden am Rotationsverdampfer bei einer Badtemperatur von 90°C und einem Vakuum von 2700 Pa eingeengt. Der Rückstand wird im Hochvakuum auf 110 bis 120°C erhitzt (Badtemperatur 140°C). Nach Eeendigung der Furan-Abspaltung steigt die Sumpftemperatur und das Produkt destilliert über. Man erhält 130 Teile N-(N',N'-Diethylaminoethyl)-acrylamid vom Siedepunkt 107°C/ 80 Pa.

**Beispiel 3:**

401,3 Teile N,N-Dimethylaminopropylamin werden mit 53 Teilen 30%iger methanolischer Na-methylatlösung versetzt. 577,5 Teile 2-Carbomethoxy-7-oxabicyclo(2,2,1)hept-5-en werden bei 20 bis 25°C unter Kühlen zugetropft. Man läßt 15 Stunden nachrühren und neutralisiert mit 17,5 Teilen konzentrierter Essigsäure. Man versetzt mit 1050 Teilen Methylenchlorid und 1050 Teilen Wasser, trennt die organische Phase ab und extrahiert die wäßrige Phase mit 1050 Teilen Methylenchlorid. Die vereinigten organischen Phasen werden am Rotationsverdampfer bei einer Badtemperatur von 90°C und einem Vakuum von 2700 Pa eingeengt. Der Rückstand wird im Hochvakuum auf 110 bis 120°C erhitzt (Badtemperatur 140°C). Nach Beendigung der Furanabspaltung steigt die Sumpftemperatur und das Produkt destilliert über. Man erhält 368 g N-(N',N'-Dimethylaminopropyl)-acrylamid von Siedepunkt 100°C/ 40 Pa.

**Beispiel 4:**

130 Teile N,N,2,2 Tetramethyl-1,3-diaminopropan werden mit 14,4 Teilen 30%iger methanolischer Na-methylatlösung versetzt. 154 Teile 2-Carbomethoxy-7-oxabicyclo(2,2,1)-hept-5-en werden bei 30 bis 35°C unter Kühlen zugetropft. Man läßt 14 Stunden nachrühren und neutralisiert mit 7,85 Teilen 50%iger Schwefelsäure. Man versetzt mit 200 Teilen Methylenchlorid und 100 Teilen Wasser, trennt die organische Phase ab und extrahiert die wäßrige Phase 3 mal mit je 50 Teilen Methylenchlorid. Die vereinigten organischen Phasen werden am Rotationsverdampfer bei einer Badtemperatur von 90°C und einem Vakuum von 2700 Pa eingeengt. Der Rückstand wird im Hochvakuum auf 110 bis 120°C erhitzt (Badtemperatur 140°C). Nach Beendigung der

Furan-Abspaltung steigt die Sumpftemperatur und das Produkt destilliert über. Man erhält 122,5 Teile N-(N',N'2',2'-Te-thylaminopropyl)acrylamid vom Siedepunkt 110° C/ 80 Pa.

**Beispiel 5:**

29,5 Teile n-Propylamin werden mit 7,2 Teilen 30%iger methamolischer Na-methylatlösung versetzt. 84 Teile 2-Car-boethoxy-7-oxabicyclo(2,2,1)hept-5-en werden bei 30° C unter Kühlen zugetropft. Man läßt 15 Stunden nachrühren, löst den ausgefallenen Kristallbrei in 100 Teilen Methylenchlorid und neutralisiert mit 4 Teilen konzentrierter Salzsäure. Man versetzt mit 50 Teilen Wasser, trennt die organische Phase ab und extrahiert die wäßrige Phase 3 mal mit je 25 Teilen Methylenchlorid. Die vereinigten organischen Phasen werden am Rotationsverdampfer bei einer Badtemperatur von 90° C und einem Vakuum von 2700 Pa eingeengt. Der Rückstand wird im Vakuum von 2700 Pa auf 110 bis 120° C erhitzt (Badtemperatur 140° C). Nach Beendigung der Furan-Abspaltung steigt die Sumpftemperatur und das Produkt destilliert bei jetzt erniederigtem Druck über. Man erhält 45 Teile N-Propylacrylamid vom Siedepunkt 88° C/ 100 Pa.

**Beispiel 6:**

134,8 Teile Stearylamin werden mit 7,2 Teilen 30%iger methanolischer Na-methylatlösung und 77 Teilen 2-Carbometh-oxy-7-oxybicyclo(2,2,1)hept-5-en versetzt. Man erhitzt auf 60° C und rührt bei dieser Temperatur 15 Stunden mach. Nun gibt man 300 Teile Chloroform zu und neutralisiert mit 4 Teilen konzentrierter Salzsäure. Man versetzt mit 100 Teilen Wasser, trennt die organische Phase ab und extrahiert die wäßrige Phase mit 50 Teilen Chloroform. Die vereinigten organischen Phasen werden am Rotationsverdampfer bei einer Badtemperatur von 90° C und einem Vakuum von 2700 Pa eingeengt. Der Rückstand wird im Hochvakuum auf 110 bis 120° C erhitzt (Badtemperatur 140° C). Nach Beendigung der Furanabspaltung steigt die Sumpftemperatur auf 140° C. Man kühlt ab, löst das Produkt heiß in Aceton, filtriert und lässt auskristallisieren. Man erhält 117 Teile N-Stearylacrylamid vom Schmelzpunkt 75 bis 77° C.

**Beispiel 7:**

64,5 Teile 2-Ethylhexylamin werden mit 7,2 Teilen 30%-iger methanolischer Na-methylatlösung versetzt. 77 Teile 2-Carbomethoxy-7-oxabicyclo(2,2,1)hept-5-en werden bei 30 bis 35° C unter Kühlen zugetropft. Man läßt 15 Stunden nachrühren und neutralisiert mit 4 Teilen konzentrierter Salzsäure. Man versetzt mit 100 Teilen Methylenchlorid und 50 Teilen Wasser, trennt die organische Phase ab und extrakiert die wäßrige Phase 2 mal mit je 25 ml Methylenchlorid. Die vereinigten organischen Phasen werden am Rotationsverdampfer bei einer Badtemperatur von 90° C und einem Vakuum von 2700 Pa eingeengt. Der Rückstand wird im Hochvakuum auf 110 bis 120° C erhitzt (Badtemperatur 140° C). Nach Beendigung der Furan-Abspaltung steigt die Sumpftemperatur und das Produkt destilliert über. Man erhält 68,6 Teile N(2-Ethylhexyl)acrylamid vom Siedepunkt 123° C/50 Pa.

**Beipseiel 8:**

43,5 Teile Morpholin werden mit 7,2 Teilen 30%iger methanolischer Na-methylat-Lösung versetzt. 77 Teile 2-Carbo-methoxy-7-oxabicyclo(2,2,1)hept-5-en werden bei 30 bis 35° C unter Kühlen zugetropft. Man läßt 15 Stunden nachrühren und neutralisiert mit 4 Teilen konzentrierter Salzsäure. Man Versetzt mit 100 Teilen Methylenchlorid und 50 Teilen Wasser, trennt die organische Phase ab und extrahiert die wäßrige Phase 4 mal mit je 25 Teilen Methylenchlorid. Die vereinigten organischen Phasen werden am Rotationsverdampfer bei einer Badtemperatur von 90° C und einem Vakuum vom 2700 Pa eingeengt. Der Rückstand wird im Vakuum von 2700 Pa auf 110 bis 120° C.erhitzt (Badtemperatur 140° C). Nach Beendigung der Furan-Abspaltung steigt die Sumpftemperatur und das Produkt destilliert bei jetzt erniedrigtem Druck über. Man erhält 48 Teile N-Morpholinoacrylamid vom Siedepunkt 87° C/130 Pa.

**Beispiel 9:**

36,5 Teile Diethylamin werden mit 7,2 Teilen 30%iger methanolischer Na-methylatlösung versetzt. 77 Teile 2-Carbomethoxy-7-oxabicyclo(2,2,1)hept-5-en werden bei 40° C zugestropft. Man läßt 15 Stunden bei dieser

Temperatur machrühren und neutralisiert mit 4 Teilen konzentrierter Salzsäure. Man versetzt mit 100 Teilen Methylenchlorid. und 50 Teilen Wasser, trennt die organische Phase ab und extrahiert die wäßrige Phase 3 mal mit je 25 Teilen Methylenchlorid. Die vereinigten organischen Phasen werden am Rotationsverdampfer bei einer Badtemperatur von 90° C und einem Vakuum von 2700 Pa eingeengt. Der Rückstand wird im Vakuum von 2700 Pa auf 110 bis 120° C erhitzt (Badtemperatur 140° C). Nach Beendigung der Furan-Abspaltung steigt die Sumpftemperatur und das Produkt destilliert bei jetzt erniedrigtem Druck über. Man erhält 21 Teile N,N-Diethylacrylamid vom Siedepunkt 55° C/130 Pa.

**Beispiel 10:**

15 Teile Ethylendiamin werden mit 7,2 Teilen 30%iger methanolischer Na-methylatlösung versetzt. 77 Teile 2-Carbomethoxy-7-oxabicyclo(2,2,1)hept-5-en werden bei 30° C len zugetropft. Mam läßt über Nachtauskristallisieren, löst mit 400 Teilen Chloroform und neutralisiert mit 4 Teilen konzentrierter Salzsäure. Man versetzt mit 50 Teilen Wasser, trennt die organische Phase ab und extrahiert die wäßrige Phase 5 mal mit je 50 Teilen Chloroform. Die vereinigten organischen Phasen werden am Rotationsverdampfer bei einer Badtemperatur von 90° C und einem Vakuum vom 2700 Pa eingeengt. Der Rückstand wird im Vakuum von 1300 Pa auf 110 bis 120° C erhitzt (Badtemperatur 140° C). Nach Beendigung der Furan-Abspaltung steigt die Sumpftemperatur auf 140° C. Man kühlt ab, löst das Produkt heiß im n-Butanol, filtriert und lässt bei 0° C auskristallisieren. Man erhält 27 g N,N'-Ethylenbisacrylamid vom Schmelzpunkt 150 bis 155° C.

**Beispiel 11:**

46,5 Teile Anilin werden mit 7,2 Teilen 30%iger methanolischer Na-methylatlösung versetzt. 77 Teile 2-Carbometh-oxy-7-oxabicyclo(2,2,1)hept-5-en werden bei 40° C zugetropft. Man läßt 15 Stunden bei dieser Temperatur machrühren und neutralisiert mit 4 Teilen konzentrierter Salzsäure. Man versetzt mit 50 Teilen Wasser, trennt die organische Phase ab. Die organische Phase wird am Rotations-Verdampfer bei einer Badtemperatur von 90° C und einem Vakuum von 2700 Pa eingeengt. Der Rückstand wird im Vakuum von 2700 Pa auf 110 bis 120° C erhitzt (Badtemperatur 140° C). Nach Beendiguns der Furan-Abspaltung steigt die Sumpftemperatur und das Produkt destilliert über. Man erhält 27,5 Teile N-Phenylacrylamid vom Siedepunkt 135° C/ 50 Pa. Das aus Acetoniltril bei -20° C umkristallisierte Produkt hat einen Schmelzpunkt von 114 bis 115° C.

**Beispiel 12:**

29,5 Teile n-Propylamin werden mit 7,2 Teilen 30%iger methanolischer Na-methylatlösung versetzt. 84 Teile 2-Carboethoxy-7-oxabicyclo(2,2,1)hept-5-en werden bei 30° C unter Kühlen zugetropft. Man läßt 15 Stunden nachrühren, löst den ausgefallenen Kristallbrei in 100 Teilen Methylen chlorid und neutralisiert mit 4 Teilen konzentrierter Salzsäure. Man versetzt mit 50 Teilen Wasser, trennt die orgamische Phase ab und extrahiert die wässrige Phase 3 mal mit je 25 Teilen Methylenchlorid. Die vereinigten organischen Phasen werden mit Magnesiumsulfat getrocknet und am Rotationsverdampfer bei einer Badtemperatur von 40° C und einem Vakuum von 2700 Pa eingeengt. Nach Zugabe von 2,2 Teilen $AlCl_3$ wird das entstehende Furan unter den gleichen Bedingungen abdestilliert. Der Rückstand wird mit 100 Teilen Methylenchlorid und 50 Teilen Wasser versetzt. Man trennt die organische Phase ab und extrahiert die wässrige Phase 3 mal mit je 25 Teilen Methylenchlorid. Die vereinigten organischen Phasen werden am Rotationsverdampfer eingeengt. Der Rückstand wird im Vakuum destilliert. Man erhält 45 Teile N-Propylacrylamid vom Siedepunkt 88° C/100 Pa.

**Patentansprüche**

1. Verfahren zur Herstellung N-substituierter Acrylsäureamide, dadurch gekennzeichnet, daß man 2-Carboalkoxy-toxabicyclo (2,2,1)hept-5-ene mit primären oder sekundären Aminen zu 2-Carboxamid-7-oxabicyclo (2,2,1)hept-5-enen umsetzt und diese, gegebenenfalls in Gegenwart von Lewis-Säuren, unter Bildung von Furan und N-substituierten Acrylsäureamiden zersetzt.

2. Verfahren nach Anspruch 1, dadurch gekennzeichnet, daß man die Umsetzung der 2-Carboalkoxy-t-oxabicyclo(2,2,1)-hept-5-ene mit den primären oder sekundären Aminen bei Temperaturen zwischen 20 und 60° C durchführt.

3. Verfahren nach einem der Ansprüche 1 bis 2, dadurch gekennzeichnet, daß man die Umsetzung der 2-Carboalkoxy-7-oxabicyclo (2,2,1)hept-5-ene mit den primären oder sekundären Aminen unter wasserfreien Bedingungen und in Gegenwart starker Basen, bevorzugt Alkalimetallalkoholaten, -hydriden oder -amiden oder

in Gegenwart von basischen Ionenaustauschern durchführt.

4. Verfahren nach einem der Ansprüche 1 bis 3, dadurch gekennzeichnet, daß man die 2-Carboxamid-7-oxabicyclo-(2,2,1)-hept-5-ene durch Erhitzen auf eine Temperatur zwischen 60 und 300, vorzugsweise zwischen 80 und 200, insbesondere zwischen 100 und 150°C zu Furan und N-substituierten Acrylsäureamiden zersetzt.

5. Verfahren nach einem der Ansprüche 1 bis 4, dadurch gekennzeichnet, daß man die Zersetzung der 2-Carboxamid-7-oxabicyc1o(2,2,1) hept-5-ene in Gegenwart von Lewis-Säuren bei einer Temperatur zwischen 0 und 40, vorzugsweise zwischen 15 und 25°C vornimmt.

6. Verfahren nach einem der Ansprüche 1 bis 5, dadurch gekennzeichnet, daß man als Lewis-Säure $AlCl_3$,$TiCl_4$, $SnCl_4$, $FeCl_3$ oder $BF_3.OET_2$ verwendet.

7. Verfahren nach einem der Ansprüche 1 bis 6, dadurch gekennzeichnet, daß man die Zersetzung der 2-Carboxamid-7-oxabicyc1o(2,2,1) hept-5-ene durch Erhitzen im Vakuum vornimmt.

8. Verfahren nach einem der Ansprüche 1 bis 7, dadurch gekennzeichnet, daß man gerad- oder verzweigtkettige aliphatische oder cycloaliphatische Amine, gegebenenfalls substituierte heterocyclische oder aromatische Amine verwendet.

9. Verfahren nach einem der Ansprüche 1 bis 8, dadurch gekennzeichnet, daß man primäre oder sekundäre Amine einsetzt, die mindestens eine weitere sekundäre oder tertiäre Aminogruppe enthalten.

## Claims

1. A process for the production of N-substituted acrylic acid amides, characterized in that a 2-carbo-alkoxy-t-oxabicyclo (2,2,1) hept-5-ene is reacted with a primary or secondary amine to a 2-carboxamide-7-oxa-bicyclo (2,2,1) hept-5-ene and decomposing said 2-carboxamide-7-oxabicyclo (2,2,1) hept-5-ene, if necessary in the presence of Lewis acids, to furane and N-substituted acrylic acid amides.

2. A process according to claim 1, characterized in that the reaction of said 2-carboalkoxy-t-oxabi-cyclo (2,2,1) hept-5-ene with said primary or secondary amines is carried out at a temperature between 20 to 60°C.

3. A process according to any one of claims 1 to 2, characterized in that the reaction of said 2-carbo-alkoxy-t-oxabicyclo (2,2,1) hept-5-ene with said primary or secondary amines is carried out under water-free conditions and in the presence of strong bases, preferably alcali metal alcoholates, -hydrides, or -amides, or basic ion exchangers.

4. A process according to any one of claims 1 to 3, characterized in that the 2-carboxamide-7-oxabicyclo (2,2,1) hept-5-ene is decomposed to furane and N-substituted acrylic acid amides by heating to a temperature between 80 to 200°C, preferably between 80 to 200°C, especiallay between 100 to 150°C.

5. A process according to any one of claims 1 to 4, characterized in that the decomposition of 2-carboxamide-7-oxabicyclo (2,2,1) hept-5-ene is carried out in the presence of Lewis acids at a temperature between 0 and 40°C, preferably between 15 and 25°C.

6. A process according to any one of claims 1 to 5, characterized in that said Lewis acid is selected from $AlCl_3$, $TiCl_4$, $SnCl_4$, $FeCl_3$ or $BF_3$. $OET_2$.

7. A process according to any one of claims 1 to 6, characterized in that the decomposition of 2-carboxamide-7-oxabicyclo (2,2,1) hept-5-ene is carried out by heating in a vacuum.

8. A process according to any one of claims 1 to 7, characterized in that said amine is a straight or branched-chain aliphatic or cycloaliphatic anine, if necessary a substituted heterocyclic or aromatic anine.

9. A process according to any one of claims 1 to 8, characterized in that said amine is a primary or secondary amine which contains at least one further secondary or tertiary amino group.

## Revendications

1. Procédé de préparation d'acryl-amides N-substitués, caractérisé en ce que l'on transforme des 2-carbalcoxy-t-oxabicyclo(2,2,1)hept-5-ènes par réaction avec des amines primaires ou secondaires en 2-carboxamido-7-oxabicyclo (2,2,1)hept-5-ènes, puis on déconpose ceux-ci, éventuellement en présence d'acides de Lewis, en furanne et acrylamides N-substitués.

2. Procédé suivant la revendication 1, caractérisé en ce que la réaction des 2-carbalcoxy-t-oxabicyclo(2,2,1) hept-5-ènes et des amines primaires ou secondaires est réalisée à des températures comprises entre 20 et 60°C.

3. Procédé suivant l'une des revendications 1 et 2, caractérisé en ce que la réaction des 2-carbalcoxy-7-oxabicyclo(2,2,1) oxabicyclo(2,2,1)hept-5-ènes et des amines primaires ou, secondaires est réalisée dans des conditions anhydres et en presence de bases fortes, de préférence d'alcoolates, hydrures ou amides de métaux alcalins, ou en présence d'échangeurs d'ions basiques.

4. Procédé suivant l'une des revendications 1 à 3, caractérisé en ce que la décomposition des 2-carbox-amido-7-oxabicyclo(2,2,1) hept-5-ènes en furanne et acryl-amides N-substitués est provoquée par leur chauffage à une température comprise entre 60 et 300°, de préférence entre 80 et 200° et plus particulièrement entre 100 et 150°.

5. Procédé suivant l'une des revendications 1 à 4, caractérisé en ce que la décomposition des 2-carboxamido-7-oxabicyclo(2,2,1)hept-5-ènes est réalisée à une température comprise entre 0 et 40° C et de préférence entre 15 et 25° C en présence d'un acide de Lewis.

6. Procédé suivant l'une des revendications 1 à 5, caractérisé en ce que l'acide de Lewis est choisi parmi $AlCl_3$, $TiCl_4$, $SnCl_4$, $FeCl_3$ et $BF_3.OEt_2$.

7. Procédé suivant l'une des revendications 1 à 6, caractérisé en ce que la décomposition des 2-carboxamido-7-oxabicyclo(2,2,1)hept-5-ènes est provoquée par un chauffage sous vide.

8. Procédé suivant l'une des revendications 1 à 7, caractérisé en ce que l'on emploie des amines aliphatiques ou cycloaliphatiques à chaîne droite ou ramifiée ou des amines aromatiques ou hétérocycliques éventuellement substituées.

9. Procédé suivant l'une des revendications 1 à 8, caractérisé en ce que l'on emploie des amines primaires ou secondaires comprenant au moins un groupe amino secondaire ou tertiaire supplémentaire.